# EUROPEAN PATENT SPECIFICATION

(11) **EP 0 781 128 B1**
(45) Date of publication and mention of the grant of the patent: **23.11.2005**
(21) Application number: 95933842.7
(22) Date of filing: 15.09.1995
(51) Int. Cl.: A61K 31/075, A61K 31/38, A61K 31/10, A61K 31/15, A61K 31/05, A61P 7/06

(54) **CLOTRIMAZOLE METABOLITES IN THE TREATMENT OF SICKLE CELL DISEASE**
CLORIMAZOLE METABOLITE IN DEN BEHANDLUNG VOM SICHELZELLANÄMIE
METABOLITES DE CLOTRIMAZOLE CONTRE LA DREPANOCYTOSE

(30) Priority: 16.09.1994 US 307874
(43) Date of publication of application: 02.07.1997
(73) Proprietor: CHILDREN'S MEDICAL CENTER CORPORATION, Boston Massachusetts 02115 (US)
(72) Inventor: BRUGNARA, Carlo, Newton, MA 02161 (US); RAFAI, Nader, Newton, MA 02167 (US); PLATT, Orah, Brookline, MA 02146 (US)
(74) Representative: Jump, Timothy John Simon
(86) International application number: PCT/US1995/011822
(87) International publication number: WO 1996/008242

(56) References cited:
- US-A- 5 273 992
- BIOCHEMICAL PHARMACOLOGY, vol. 41, no. 12, 1991 pages 1949-56, MATSUURA ET AL 'Structure-activity relationships in the induction of hepatic microsomal cytochrome P450 by clotrimazole and its structurally related compounds in rats'
- J. PHARMACOL EXP THER, vol. 273, no. 1, 1995 pages 266-73, BRUGNARA ET AL 'Oral administration of clotrimazole and blockade of human erythrocyte Ca++-activated K+ channel: the imidazole ring is not required for inhibitory activity'
- DATABASE WPI Section Ch, Week 8142 Derwent Publications Ltd., London, GB; Class B05, AN 81-76452D & JP-A-56 110 615 ( ITOTA H) , 1 September 1981
- ACTA PHYTOPATHOLOGICA ACADEMIAE SCIENTIARUM HUNGARICAE, vol. 13, no. 1-2, 1978 pages 227-34, LUKOVITS ET AL 'Quantitative relationship between structure and anti-ecdysone activity of triarimol analogues'

## Description

### Background of the Invention

Sickle Cell Anemia is a disease which is characterized by the presence of thin sickle-shaped and crescent-shaped red blood cells. The sickle shape is a result of an inherited mutation in hemoglobin. The sickled hemoglobin (Hb S) tends to aggregate into rod like structures when the cell is in a deoxygenated state. The formation of these rod-like structures gives the cell its sickled appearance. The red blood cells which have become sickled due to the mutant hemoglobin are fragile and are more susceptible to breakage, which results in anemia.

The molecular abnormality associated with the mutant hemoglobin has been determined to be the consequence of a single nucleotide substitution (Marotta et al., J. Biol. Chem. 252: 5040-5053, 1977). As a result of the nucleotide substitution, a valine residue is inserted rather than a glutamic acid residue at the number 6 position of the beta globin chain (Ingram, Nature 178: 792-794, 1956). Under conditions of low oxygen, substitution with a hydrophobic valine results in the formation of a pocket at the corner of the beta chain of the Hb S. The hydrophobic residue causes the Hb S molecule to fit easily into the end of another hemoglobin molecule, allowing for the Hb S molecules to interact with one another to form rod-like structures. The nature of the molecular arrangement of Hb S subunits in the oxygenated form prevents the formation of rods under conditions of oxygen abundance.

Water comprises approximately 70% of the weight of a normal red blood cell. When water is lost from the cell there is an increase in cytoplasmic viscosity as the mean cell hemoglobin concentration (MCHC) rises above about 32g/dl. Because changes in the hydration state of the cell influence changes in the concentration ofHb S, dehydration of sickle cells is an essential step in the pathophysiology of sickle cell disease. Small increases in cell Hb S concentration dramatically enhance Hb S polymerization and cell sickling.

Red blood cells contain a potassium channel, the Gardos channel, which is a member of the large family of calcium-activated potassium channels. The movement of potassium, chlorine, and water across the cell membrane results from the activation of the Gardos channel upon changes in the intracellular free calcium concentration. The Gardos channel plays a major role in the dehydration of sickle cells and therefore the study of its activation is important to achieving a complete understanding of the mechanisms of sickle cell anemia.

ChTX, a small peptide of 37 amino acids in length, is a potent inhibitor of the Gardos channel as well as many other Ca²⁺ activated K channels. Because bound inhibitor may be displaced by the binding of activators to the channel, ChTX is an important tool for the study of Gardos channel function and activation.

Several strategies have been followed in an attempt to treat sickle cell anemia. A variety of approaches have attempted to decrease the polymerization of Hb S by lowering the osmolality of plasma. These strategies have included the induction of hyponatremia and cell swelling by DDAVP, decreasing Hb S concentration by increasing Hb F concentration using hydroxyurea and butyrate derivatives, intravenous infusion of distilled water, intravenous infusion of hypotonic saline, administration of the anti-diuretic hormone vasopressine, the use of monensin to increase cation content of the sickle cell, intravenous administration of cetiedil, and use of oxypentofyllin. Most of these studies have proved to be impractical as either prophylactic or therapeutic treatment regimens, because of high toxicity and/or side effects which outweigh the benefits and value of using them.

Another approach to the prevention of sickle cell dehydration involves the inhibition of potassium transport in erythrocytes. By inhibiting the loss of potassium from the cell, dehydration is prevented. U.S. patent 5,273,992 describes the use of imidazoles and nitroimidazoles to inhibit the loss of potassium via the Gardos channel. Clotrimazole (CLT), an imidazole, has been shown to be a specific inhibitor of the Gardos channel. (Brugnara et al., 1993). When combined with a compound which stabilizes the oxyconformation of Hb S, CLT induces an additive reduction in the clogging rate of a micropore filter and may attenuate the formation of irreversibly sickled cells (Stuart et al., 1994). The group of imidazoles believed to be useful includes CLT, miconazole, econazole, butoconazole, oxiconazole and sulconazole.

In U.S. Patent 5,273,992 as well as other unpublished studies it has been found that such compounds with an imidazole group have the ability to regulate Ca⁺⁺activated potassium channels. It is the imidazole group which is common to all of these active compounds and it was believed that the imidazole group was a component of the functionally active part of the compound.

### SUMMARY OF THE INVENTION

The present invention provides the use of compounds in the manufacture of a medicament for the treatment of sickle cell disease. The present invention also provides methods for reducing sickle erythrocyte dehydration and delaying the occurrence of erythrocyte sickling. It has been discovered that the imidazole functionality of the compounds mentioned in the Background of the Invention is not required for the therapeutic activity of such compounds.

According to one aspect of the invention, there is provided the use of a compound having the general formula: 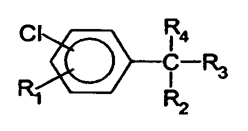 wherein R1 is selected from the group consisting of hydrogen, hydroxyl and halogen; wherein R2 is selected from the group consisting of nothing, hydrogen, phenyl and substituted phenyl, the substitution being a hydroxyl group; wherein R3 is selected from the group consisting of hydrogen, hydroxyl, lower alkyl, and lower alkoxy; wherein R4 is selected from the group consisting of sulfur-CH₂-R5, oxygen-CH₂-R5,=N-O-CH₂-R5, oxygen-phenyl-CH=CH₂, CH₂-C(CH₃)H-S-substituted phenyl, phenyl, and substituted phenyl, the substitution being selected from the group consisting of a hydroxyl group and a halogen; wherein R5 is selected from the group consisting of vinyl, phenyl, halogen mono-substituted phenyl, halogen di-substituted phenyl, phenyl-S-phenyl, CH₂-O-phenyl, CH₂-O-(halogen substituted)phenyl, and: 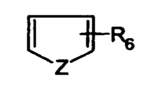 (wherein Z is sulfur, oxygen, or nitrogen); and R6 is selected from the group consisting of hydrogen and halogen, in the manufacture of a medicament for the treatment of sickle cell disease.

According to a second aspect of the present invention, a method for reducing sickle erythrocyte dehydration and for delaying the occurrence of erythrocyte sickling is provided, whereby a compound as defined in the first aspect of the invention is administered *ex vivo* or *in vitro,* and the administered compound is allowed to induce inhibition of the Ca²⁺ -activated potassium channel at the membranes of the sickle erythrocytes such that sickle erythrocyte dehydration is reduced and the occurrence of erythrocyte sickling is delayed.

### BRIEF DESCRIPTION OF THE DRAWINGS:

The present invention may be more easily and completely understood when taken in conjunction with the accompanying drawings, in which:
FIG. 1 is a representative chromatogram for plasma collected 4 hours following oral administration of CLT in a subject;
FIG. 2 is a diagram of the major metabolic structures of CLT;
FIG. 3 is a graph depicting the plasma levels of CLT and the two major CLT metabolites following oral administration of 1 gram of CLT;
FIG. 4 is a graph depicting the inhibition of Ca-activated ⁸⁶Rb influx by oral administration of CLT as a function of time following oral administration of 1 gram of CLT;
FIG. 5 is a graph representing the relationship between summed plasma levels of CLT, metabolite A and B and the inhibition of Ca²⁺ activated ⁸⁶Rb influx measured in whole blood in each of four subjects;
FIG. 6 is a graph depicting the specific binding of metabolite B as a function of displacement of ¹²⁵I-ChTX;
FIG. 7 is a graph depicting the relationship between concentration of metabolite B and the inhibition of Ca²⁺ activated ⁸⁶Rb influx measured in whole blood.

### DETAILED DESCRIPTION OF THE INVENTION

The invention involves the reduction in sickle cell dehydration such as in the treatment of sickle cell disease. It has previously been established in US Patent No. 5,273,992 that administration of imidazoles and nitroimidazoles to subjects suffering from sickle cell disease results in a reduction in erythrocyte dehydration and delays the occurrence of erythrocyte sickling. The present invention involves the unexpected finding that metabolites and analogs of these imidazoles can accomplish a reduction in erythrocyte dehydration and resultant inhibition of erythrocyte sickling, despite the fact that they do not contain an imidazole or nitroimidazole group.

The molecules useful according to the invention are of the following general formula 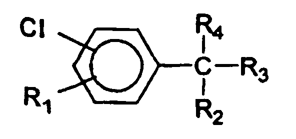 wherein R1 is selected from the group consisting of hydrogen, hydroxyl and halogen; wherein R2 is selected from the group consisting of nothing, hydrogen, phenyl, and substituted phenyl, the substitution being a hydroxyl group; wherein R3 is selected from the group consisting of hydrogen, hydroxyl lower alkyl, and lower alkoxy; wherein R4 is selected from the group consisting of sulfur-CH₂-R5, oxygen-CH₂-R5, =N-O-CH₂-R5, oxygen-phenyl-CH=CH₂, CH₂-CH-S-substituted phenyl, phenyl and substituted phenyl, the substitution being selected from the group consisting of a hydroxyl group and a halogen; wherein R5 is selected from the group consisting of vinyl, phenyl, halogen mono-substituted phenyl, halogen di-substituted phenyl, phenyi-S-phenyl. CH₂-O-phenyl, CH₂-O-(halogen substituted) phenyl, and: 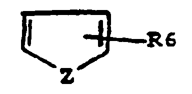 (wherein Z is sulfur, oxygen, or nitrogen); and R6 is selected from the group consisting of hydrogen and halogen.

The most preferred compound is 2-chlorophenyl-bis-phenyl-methanol. It is a metabolite of CLT and has the following chemical formula.

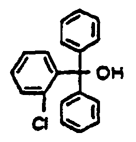

Other compounds believed useful according to the invention include the following other derivatives of clotrimazole and derivatives of other imidazoles.

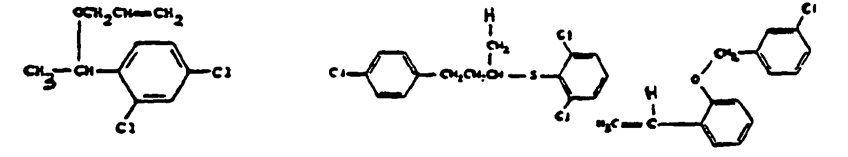

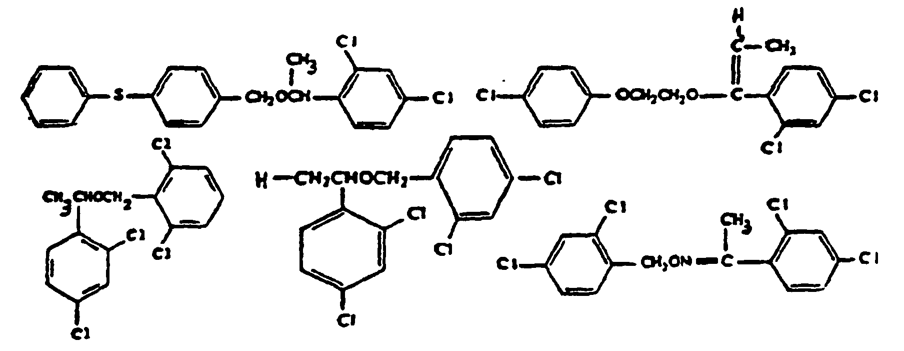

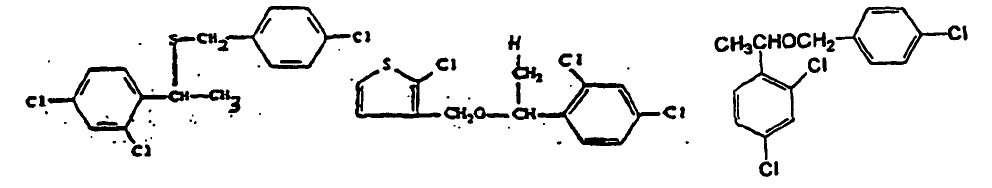

The foregoing compounds are derived from commercially available imidazoles or synthetized de novo using routine chemical synthetic procedures known to those of ordinary skill in the art.

The present invention is useful whenever it is desirable to reduce sickle erythrocyte dehydration and delay the occurrence of sickle erythrocyte distortion in-situ, and desirably in the microcirculation of a human afflicted with sickle cell disease. By definition, the word "in-situ" encompasses and includes the terms "in-vivo", "ex-vivo" and "in-vitro".

The invention is useful, inter alia, whenever it is desirable to diminish the time and duration of red cell sickling and vaso-occlusion in the blood circulation. This includes treating sickle cell disease prophylactically to decrease hemoglobin S concentration and prevent sickling as well as therapeutically treating patients with acute sickle cell crisis by intramuscular or intravenous administration. The invention may also be used for the treatment of chronic sickle cell episodes for example to control the frequency and/or duration of the episodes.

When administered, the formulations of the invention are applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthalene-2-sulphonic, and benzene sulphonic. Also, pharmaceutically acceptable salts can be prepared as alkyline metal or alkyline earth salts, such as sodium, potassium or calcium salts of the carboxylic acid group.

Suitable buffering agents include: acetic acid and a salt (1-2% W/V); citric acid and a salt (1-3% W/V); boric acid and a salt (0.5-2.5% W/V); and phosphoric acid and a salt (0.8-2% W/V).

Suitable preservatives include benzalkonium chloride (0.003-0.03% W/V); chlorobutanol (0.3-0.9% W/V); parabens (0.01-0.25% W/V) and thimerosal (0.004-0.02% W/V).

The active compounds of the present invention may be a pharmaceutical composition having a therapeutically effective amount of an imidazole metabolite optionally included in a pharmaceutically-acceptable carrier. The term "pharmaceutically-acceptable carrier" as used herein means one or more compatible solid or liquid filler, dilutants or encapsulating substances which are suitable for administration to a human or other animal. The term "carrier" denotes an organic or inorganic ingredient, natural or synthetic, with which the active ingredient is combined to facilitate the application. The components of the pharmaceutical compositions also are capable of being comingled with the molecules of the present invention, and with each other, in a manner such that there is no interaction which would substantially impair the desired pharmaceutical efficacy.

Compositions suitable for parenteral administration conveniently comprise a sterile aqueous preparation of the active compound, which is preferably isotonic with the blood of the recipient. This aqueous preparation may be formulated according to known methods using those suitable dispersing or wetting agents and suspending agents. The sterile injectable preparation may also be a sterile injectable solution or suspension in a non-toxic parenterally-acceptable diluent or solvent, for example as a solution in 1, 3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic monoor di-glycerides. In addition, fatty acids such as oleic acid find use in the preparation of injectables. Carrier formulations suitable for oral, subcutaneous, intravenous, intramuscular, etc. can be found in Remington's Pharmaceutical Sciences, Mack Publishing Company, Easton, PA.

The imidazole metabolites useful in the invention may be delivered in a mixture with other anti-sickle cell drugs including any of imidazoles described in U.S. patent 5,273,992, the entire disclosure of which is incorporated herein by reference. The '992 patent describes imidazoles, nitroimidazoles and triazoles for the treatment of sickle cells in-situ. Imidazoles include clotrimazole, miconazole, ketaconazole, econazole, butoconazole, oxiconazole, sulconazole, teoconazole. Triazole compounds include fluconazole, terconazole and intraconaloze. Nitroimidazole compounds include metronidazole, tinidazole, nimorazole, ornidazole and benznidazole. In this embodiment, a common administration vehicle (e.g., pill, tablet, implant, injectable solution, etc.) would contain both the imidazole metabolite useful in this invention and the anti-sickle cell drug and/or supplementary potentiating agent. Thus, the present invention also provides pharmaceutical compositions, for medical use, which comprise the active compounds of the invention together with one or more pharmaceutically acceptable carriers thereof and optionally any other therapeutic ingredients.

The formulations of the invention are administered in effective amounts. An effective amount is one sufficient to inhibit the calcium-activated potassium channel of erythrocytes, thereby effectively lowering the hemoglobin S concentration (and thus intracellular polymerization), thereby resulting in a decrease in erythrocyte sickling. Effective amounts will depend, of course, on the particular condition being treated; the severity of the condition; individual patient parameters including age, physical condition, size and weight; concurrent treatment; frequency of treatment; and the mode of administration. These factors are well known to those of ordinary skill in the art and can be addressed with no more than routine experimentation. It is preferred generally that a maximum dose be used, that is, the highest safe dose according to sound medical judgment, particularly if acute sickle cell crises are the dominant clinical manifestation.

Dosages are determined based on the measured inhibition of the Ca-activated K channel of erythrocytes. Target plasma levels will be those capable of inducing 75% inhibition. Dosage may be adjusted appropriately to achieve desired drug plasma levels. Generally, daily oral doses of active compounds will be from about 0.01 milligrams/kg per day to 1000 milligrams/kg per day. It is expected that oral doses in the range of 50 to 500 milligrams/kg, in one or several administrations per day, will yield the desired results. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds.

A variety of administration routes are available. The particular mode selected will depend of course, upon the particular drug selected, the severity of the sickle disease state being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, topical, nasal, transdermal or parenteral routes. The term "parenteral" includes subcutaneous, intravenous, intramuscular, or infusion. Intravenous and intramuscular routes are not particularly suited for long term therapy and prophylaxis. They could, however, be preferred in emergency situations. Oral administration will be preferred for prophylactic treatment because of the convenience to the patient as well as the dosing schedule.

The compositions may conveniently be presented in unit dosage form and may be prepared by any of the methods well known in the art of pharmacy. All methods include the step of bringing the active compounds into association with a carrier which constitutes one or more accessory ingredients. In general, the compositions are prepared by uniformly and intimately bringing the active compounds into association with a liquid carrier, a finely divided solid carrier, or both, and then, if necessary, shaping the product.

Compositions suitable for oral administration may be presented as discrete units such as capsules, cachettes, tablets, or lozenges, each containing a predetermined amount of the active compound. Other compositions include suspensions in aqueous liquors or non-aqueous liquids such as a syrup, an elixir, or an emulsion.

Other delivery systems can include time-release, delayed release or sustained release delivery systems. Such systems can avoid repeated administrations of the active compounds of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, diand triglycerides; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775 (Kent); 4,667,014 (Nestor et al.); and 4,748,034 and 5,239,660 (Leonard) and (b) diffusional systems in which an active component permeates at a controlled rate through a polymer, found in U.S. Patent Nos. 3,832,253 (Higuchi et al.) and 3,854,480 (Zaffaroni). In addition, a pump-based hardware delivery system can be used, some of which are adapted for implantation.

Use of a long-term sustained release implant may be particularly suitable for treatment of chronic sickle cell disease. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above.

### EXAMPLE 1

### MEASUREMENT OF CLT METABOLITE LEVELS FOLLOWING ADMINISTRATION OF CLT

### Study design:

Four normal healthy volunteers, three males and one female, ages 39(A) 38(B) 44(C), and 45(D), respectively, were selected for this study. Venous blood was obtained for base line measurements of red cell ion transport. The following morning, following a standardized breakfast (2 eggs, 1 slice of toast and 1 cup of coffee), each subject ingested 1 gram of CLT (Mycelex, 500 mg; Miles Pharmaceuticals, West Haven, CT). Venous blood was sampled at 2, 4, 6, 8, 10, 24 and 34 hours following the ingestion of CLT.

### Reverse Phase HPLC Assay for the Determination of CLT Levels in Blood:

CLT Standards, at concentrations of 1.0, 2.0 and 6.0 *µ*mol/L were prepared by adding to filtered serum appropriate volumes of CLT stock solution in methanol (100*µ*mol/L). 50*µ*L of the internal standard, testosterone propionate (100*µ*mol/L), and 2mL of 15% acetonitrile were added to 200*µ*L patient sera, to standards, and to controls, and then vortexed for 30 seconds. Mixtures were applied to C-18 columns (Bond-Elut; 100mg bed-size) which were preconditioned by sequential additions of 2mL of 95% ethanol and 2mL of 15% acetonitrile. Columns were washed with 9mL of 30% acetonitrile and drugs were eluted, after drying the column with 200*µ*L of 95% ethanol. 10*µ*L of each eluate was injected into the HPLC [Waters Associates System-LC-8DB, 50m X 4.6 mm column from Supelco; Mobile phase mixture of potassium phosphate dibasic (25 mmol/L) /potassium phosphate monobasic (30mmol/L)/ ethanol at a ratio of 262/13/725, respectively; flow rate of 1.0 ml/min; detector wavelength at 210 nm]. The retention times for CLT and the testosterone internal standard were 2.6 and 3.7 min., respectively. The assay was linear between 0.1 and 10*µ*mol/L of CLT, with 100.9% recovery. Run-to-run precision for concentration of 1.0*µ*mol/L and 5.0*µ*mol/L were 3.2% and 2.1%, respectively.

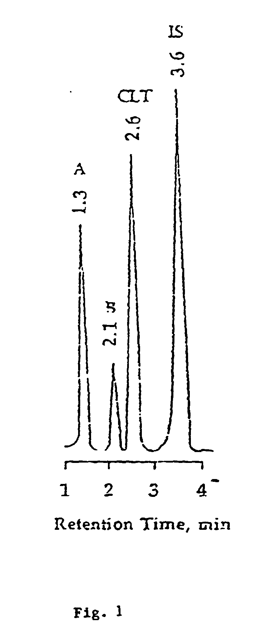

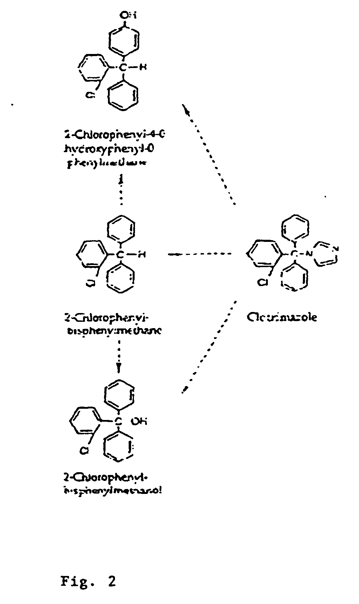

Figure 1 shows a typical chromatogram of the HPLC assay of the plasma CLT and CLT metabolite levels for one of the subjects studied, four hours after CLT administration. The third peak on the chromatogram represents CLT, which eluted at 2.6 minutes. Peak #4 represents the internal standard. The two additional peaks present displayed shorter elution times than that of the CLT, and represent the presence of two major CLT metabolites. The same two peaks, but not the CLT peak were present in urine samples collected 4 hours after CLT administration in subjects C and D (not shown). The major metabolite of CLT has previously been identified as 2-chlorophenyl-4-hydroxyphenyl-phenyl-methane, accompanied by lesser amounts of 2-chlorophenyl-bis-phenyl-methane and 2-chlorophenyl-bis-phenyl-methanol (O-chlorophenyl-diphenyl methanol), the structures of which are presented in Fig. 2. 2-chlorophenyl-bis-phenyl-methanol elutes with a similar retention time to the peak identified as metabolite B (2.1 min).

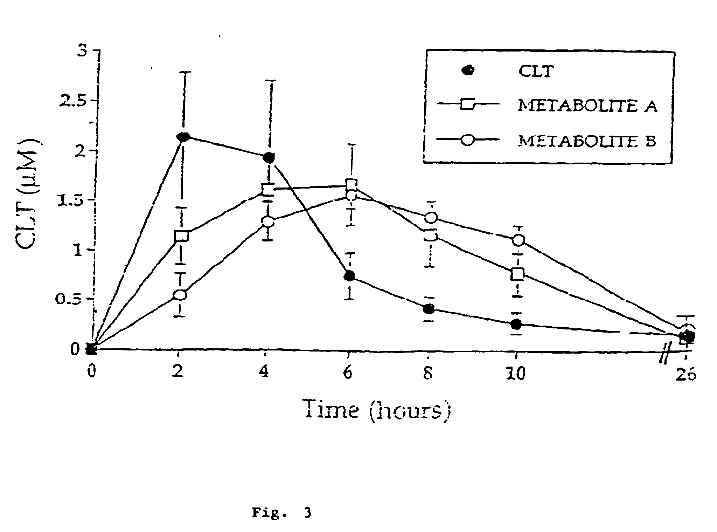

Fig. 3 shows changes in plasma levels of CLT and its two metabolites as a function of time following single 1g oral doses of CLT in the four subjects. CLT plasma levels varied considerably among the four subjects tested. Maximum attained levels ranged from 3.3*µ*M (subject D, at 4 hours) to 0.3*µ*M (subject B, at 2 hours) but peaked in all subjects between 2 and 4 hours after ingestion. Levels of metabolite B peaked at 4-6 hours with nominal maximum concentrations ranging from 1.9*µ*M (subject C, at 6 hours) to 1.6*µ*M (A and D at 6 hours, B at 4 hours). Metabolite A levels also peaked at 6 hours with maximum nominal values ranging from 2.6 *µ*M(subject D, at 6 hours) to 0.8 *µ*M (subject B, at 4 hours). Two subjects had no detectable CLT levels at 26 hours (A and B), whereas subject D had levels of 0.5*µ*M at 34 hours and no drug detectable at 100 hours. Subject C had plasma levels of 0.2*µ*M still present after 100 hours. There were no traces of the two metabolites in the plasma of any subject 100 hours following CLT administration.

### EXAMPLE 2

### THE ROLE OF CLT METABOLITES IN HUMAN RED CELL CA²⁺-ACTIVATED POTASSIUM TRANSPORT

### Study design:

The study was conducted as described in Example 1. Inhibition of red cell calcium-activated-potassium transport was measured at base line, 2, 4, 6, 8, 10, and 26 hours after CLT administration.

### Measurement of ⁸⁶Rb influx in whole blood

Three 1 ml aliquots of blood collected in Na-Heparin were incubated for 60 minutes at room temperature in 1.5 ml Eppendorf tubes in the presence of A23187 (80*µ*mol/L blood) and tris-3-(N-morpholino) propanesulfonic acid (MOPS; 20 mM in plasma). Tubes were wrapped in aluminum foil to prevent inactivation of A23187 by light, and gently rotated (15 rotations per min.). At the end of the incubation, 100*µ*L of blood was spun through 0.5mL M-butylphthalate and an aliquot of plasma was taken for measurement of potassium concentration by atomic absorption. The remaining 0.9 ml of blood was centrifuged at 6000 rpm for 10 seconds in a microfuge. ⁸⁶Rb (2*µ*cCi/ml blood) was added to the supernatant, and the cell pellets were resuspended to initiate influxes. At specified time intervals (1 and 3 minutes), triplicate 0.1 ml aliquots were taken and spun through 0.8 ml of saline medium with 15 mM ethylene glycol tetra-acetic-acid (EGTA), pH 7.4, layered over a 0.4 ml cushion of N-Butylphthalate. After aspiration of the supernatant and upper layer of oil, the remaining tube contents were frozen in acetone/dry ice, the tube tip containing the cell pellet was cut off, and the red cell-associated radioactivity was counted in a gamma counter (Micromedics 2000 HE). At the three minute time point, the supernatant was removed for determination of specific activity. Preliminary experiments indicated no significant changes in the external potassium concentration between 0 and 3 minutes. Flux calculations and experiments in washed cells suspended in saline were carried out as described (see Brugnara et al., 1993b). At base line, before CLT administration, similar values of calcium-activated ⁸⁶Rb influx were obtained for whole blood and cells washed and resuspended in saline.

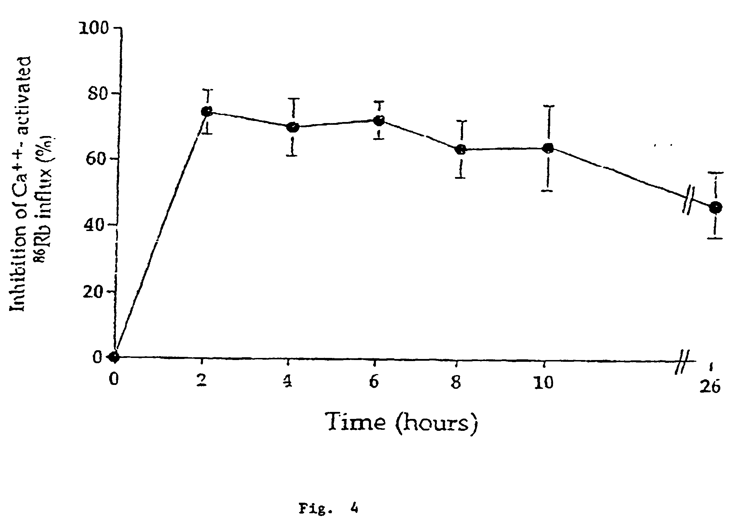

Fig. 4 shows the inhibition of calcium-activated potassium transport measured in whole blood of four normal subjects after the ingestion of a standard dose of CLT. There was marked inhibition of transport in all four subjects studied, which persisted up to 36 hours following CLT ingestion. In the four subjects tested, repeated measurements over the first 10 hours showed inhibition ranging from a low of 45% (subject B, at 8 hours) to a high of 92% (subject C, at 4 hours). In blood from subjects A and B, no residual inhibition of transport was present 34 hours after CLT administration. Red cells from subjects C and D displayed 28% and 67% inhibition at 34 hours, respectively, but had no residual inhibition at 100 hours.

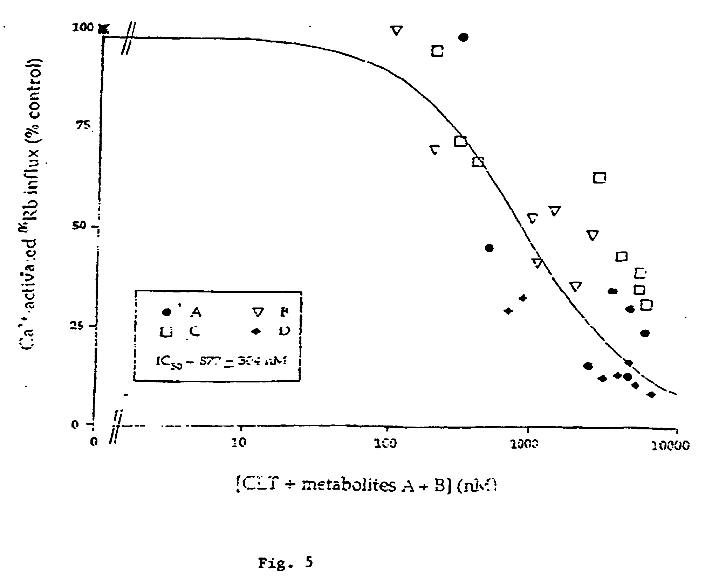

When all of the data from the four subjects studied were pooled, a significant correlation was observed between the measured inhibition of red cell calcium-activated potassium transport and the summed levels of CLT and the two major CLT metabolites [% inhibition = 42.1 log (CLT+Met A+B, *µ*M) + 48.2; r² = 0.755, t = 10.2 p < 0.001, n=36]. This inhibition exhibited an apparent IC₅₀ value of 0.88 +/-0.3 *µ*M (Fig. 5). A significant relationship was also noted between plasma levels of CLT and percent inhibition [% inhibition = 28.9 log (CLT, *µ*M) + 71.4; r² = 0.402, t = 4.78, p < 0.001, n=36], but transport inhibition was correlated best with the summed plasma levels of CLT and its metabolites. These data suggest that at least one CLT metabolite itself has inhibitory activity on the red cell Gardos channel, and that CLT perhaps partitions into the red cell.

### EXAMPLE 3

### MEASUREMENT OF DISPLACEMENT OF ¹²⁵IChTX BY METABOLITE B

### ¹²⁵IChTx binding to red cells

White cells were removed by passing 0.8mL of packed red cells through a 5 mL syringe containing a mixture of equal parts of alfa-cellulose and microcrystalline cellulose as originally described by Beutler and West (J. Lab. Clin. Med. 88: 328-333 (1976)). Red cells were washed three times in binding medium containing 18mM NaCl, 2mM KCl, 10mM tris-Cl, pH 8.0, 230mM sucrose and 0.25% bovine serum albumin. A suspension was then made in the same medium at 15% HCt. Cells were added to 3.5ml of binding medium containing ¹²⁵IChTX to a final concentration of 1 X 10⁷ cells/ml, in the absence or presence of the specified drugs. Tubes containing cell suspension were gently rotated for 90 min. at room temperature. At the end of the incubation, aliquots of 1ml were pelleted by microfuge and washed 3 times at 4°C with a solution containing 200mM NaCl, 10mM tris-Cl, pH 8.0. The washed red cell pellet was then lysed in 1 ml of 0.01% Acitionox, and counted in a gamma counter. Aliquots of binding medium were counted prior to addition of cells and at the end of the binding assay.

### Displacement by Metabolite B

Metabolite B was added to the red cells from a stock solution in acetonitrile. Similar amounts of acetonitrile were added to the control tubes. Specific binding was estimated on the basis of displacement of ¹²⁵I-ChTX by 50nM unlabled ChTX. Various concentrations of metabolite B or unlabled ChTX were added to the cell suspension (1×10⁷ cells/ml) prior to the addition of ¹²⁵I-ChTX.

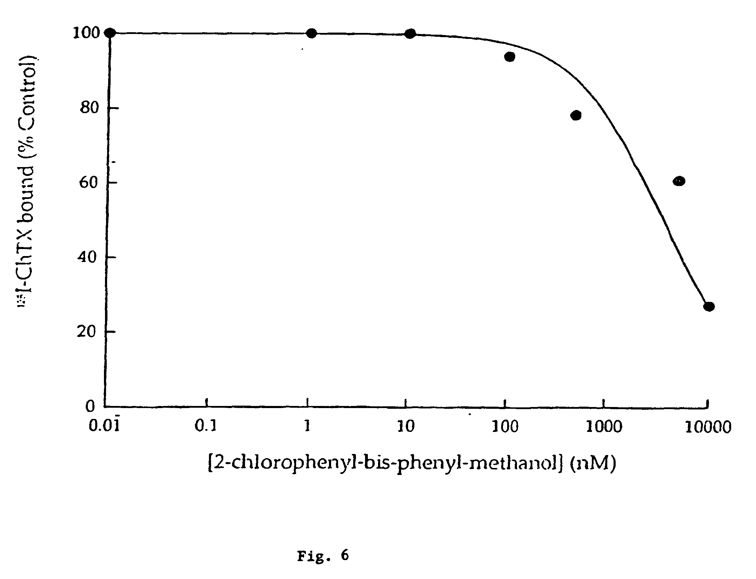

The results of the assay are depicted in Table 1. Fig. 6 represents a plot of the specific binding of metabolite B. Metabolite B specifically displaces ¹²⁵IChTX binding.

**TABLE 1**

| | ¹²⁵I-ChTX binding | SD | % displacement | SD |
|---|---|---|---|---|
| control | 1.279 | 0.03 | 0.0% | 2.3% |
| 50 *µ*M chTX | 0.608 | 0.002 | 100.0% | 0.3% |
| 10 *µ*M CLT | 1 | 0.06 | 41.6% | 6.0% |
| 1 nM Metabolite | 1.403 | 0.13 | -18.5% | 9.3% |
| 10 nM Metabolite | 1.399 | 0.15 | -17.9% | 10.7% |
| 100 nM Metabolite | 1.239 | 0.35 | 6.0% | 28.1% |
| 500 nM Metabolite | 1.134 | 0.07 | 21.6% | 6.1% |
| 1 *µ*M Metabolite | 1.327 | 0.05 | -7.2% | 4.1% |
| 5 *µ*M Metabolite | 1.016 | 0.07 | 39.2% | 7.0% |
| 10 *µ*M Metabolite | 0.79 | 0.04 | 72.9% | 5.1% |

### EXAMPLE 4

### THE EFFECTS OF CLT AND METABOLITES ON GARDOS CHANNEL FUNCTION AFTER REPEATED DOSFS OF CLT

### Study Design:

Two subjects, one male (A, 72Kg) and one female (D, 56Kg), ingested one 500mg CLT tablet every twelve hours for six days, corresponding to a daily CLT dose of 14 and 18 mg/kg, respectively. Blood was collected six hours after the AM dose of CLT on days 3 and 6 for measurement of inhibition of red cell calcium-activated potassium transport.

Table 2 depicts the results of the measurement of the red cell calcium-activated potassium transport in the two normal subjects. As shown in Table 2, there was significant inhibition of calcium-activated potassium transport during CLT administration, which persisted for at least 7 days after the drugs was discontinued. At day 6, plasma CLT concentration was 0.2*µ*M with combined metabolite levels of 2.9 *µ*M (subject A) and 3.85 *µ*M (subject D). There was no evidence of CLT or CLT-metabolites in plasma two days after CLT administration was stopped.

**TABLE 2**

| EFFECT OF 6-DAY COURSE OF ORAL CLT ON CLT BLOOD LEVELS AND RED CELLS Ca²⁺-ACTIVATED K⁺ TRANSPORT IN TWO NORMAL SUBJECTS | | | | | | |
|---|---|---|---|---|---|---|
| | CLT *µ*M | CLT Levels Plasma Met-B *µ*M | Met-A *µ*M | Blood Cells CLT *µ*mol/L cells | Met A+B | Ca²⁺ -activated ⁸⁶Rb influx inhibition % |
| Subject A Baseline | 0 | 0 | 0 | 0 | 0 | 0±4 |
| CLT day 3 | 0.2 | 1.1 | 0.7 | 0.45 | 4.45 | 74±5 |
| CLT day 6 | 0.2 | 1.1 | 1.8 | 0.8 | 4.15 | 73±4 |
| Wash-out day 6 | 0 | <0.1 | <0.1 | 0.4 | 0 | 71±7 |
| Wash-out day 10 | 0 | <0.1 | <0.1 | ND | 0 | 75±11 |
| Wash-out day 13 | 0 | 0 | 0 | 0.85 | 0 | 58±9 |
| Wash-out day 20 | 0 | 0 | 0 | 0 | 0 | 0±10 |
| Subject B Baseline | 0 | 0 | 0 | 0 | 0 | 0±10 |
| CLT day 3 | 0.25 | 0.8 | 1.65 | 0.2 | 4.2 | 80±28 |
| CLT day 6 | 0.2 | 1.35 | 2.5 | 1.1 | 7.3 | 83±7 |
| Wash-out day 8 | 0 | <0.1 | <0.1 | 0.6 | 0 | 79±21 |
| Wash-out day 10 | 0 | <0.1 | <0.1 | 0.65 | 0 | 82±4 |
| Wash-out day 13 | 0 | 0 | 0 | 0.7 | 0 | 37+30 |
| Wash-out day 20 | 0 | 0 | 0 | 0.25 | 0 | 0±13 |

Measurements of whole blood CLT levels allowed estimation of "blood cell-associated" CLT levels. As shown in Table 2, significant levels of "blood cell-associated CLT were detected up to 7 days (subject A) and 14 days (subject D) following CLT withdrawal. There were no metabolites detectable in blood cells 2 days after CLT was discontinued. These data suggest that red cells and possibly other blood elements bind or contain a significant amount of CLT for an extended period of time, even in the absence of measurable plasma levels. CLT metabolites show a different behavior, disappearing at the same time from both plasma and cells.

There was a significant correlation between summed levels of CLT and its metabolites in cells and the percent inhibition of the Gardos channel measured in whole blood [% inhibition = 31.7 log (CLT + Met A + Met B, *µ*M) + 56.4; r²=0.439, t = 3.06, p < 0.02, n=14].

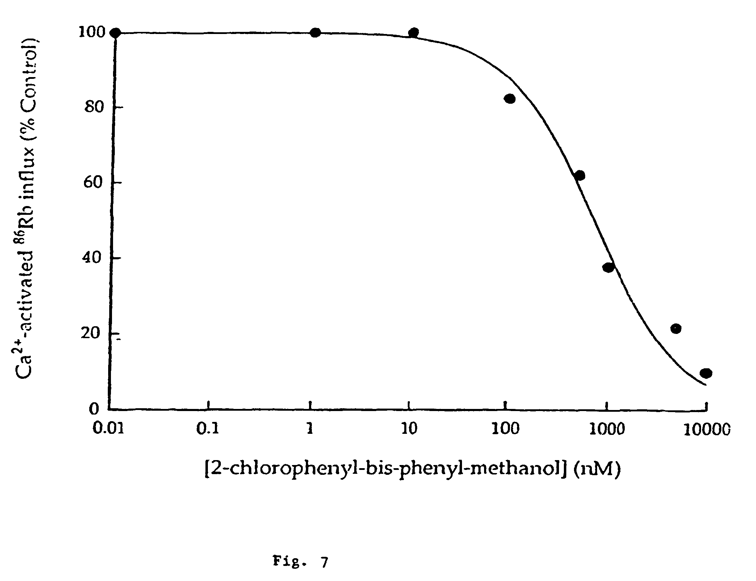

Fig. 7 shows the inhibition of calcium-activated potassium transport by metabolite B in comparison with CLT. Metabolite B specifically inhibits potassium transport via the red cell Gardos channel. CLT and metabolite B were incubated with a red cell suspension at 20% Hct. Comparison of the inhibitory effect on the red cell Gardos channel of CLT and metabolite B shows IC₅₀ values of 310 +/- 63nM for CLT and 720 +/- 190nM for metabolite B. The value for the inhibition of K transport by metabolite B is two to three fold lower than the IC₅₀ for displacement of ¹²⁵I-ChTX by metabolite B. It has previously been shown, (Brugnara, J. Biol. Chem. 1993) for ChTX that there is a two to three fold increase in the IC₅₀ value for displacement of ¹²⁵I-ChTX by ChTX compared with the inhibition ofK transport by ChTX.

Inhibition of K transport was measured at varying concentrations of metabolite B and CLT, and the results are depicted in Table 3. The percent inhibition of K transport was greater than 50% when the cells were treated with 500nM CLT or 1*µ*M metabolite B and reached maximal levels at 5*µ*M CLT and 10*µ*M metabolite B.

Oral administration of CLT was not associated with significant side effects in any of the subjects studied. In particular no nausea, vomiting or diarrhea were observed. No changes were observed in liver function tests, plasma creatine or BUN.

**TABLE 3**

| **[drug]** | **flux** | **% control** | **% inhib.** |
|---|---|---|---|
| | | | |
| Control | 1.152 | 100.0% | 0.0% |
| | | | |

| **CLT:** | | | |
|---|---|---|---|
| 1 nM | 1.155 | 100.3% | -0.3% |
| 10 nM | 1.124 | 97.6% | 2.4% |
| 100 nM | 0.892 | 77.4% | 22.6% |
| 500 nM | 0.518 | 45.0% | 55.0% |
| 1 µM | 0.248 | 21.5% | 78.5% |
| 5 µM | 0.038 | 3.3% | 96.7% |
| 10 µM | 0.043 | 3.7% | 96.3% |
| | | | |

| **2-chlorophenyl-bis-phenyl-methanol** : | | | |
|---|---|---|---|
| 1 nM | 1.444 | 125.3% | -25.3% |
| 10 nM | 1.115 | 96.8% | 3.2% |
| 100 nM | 0.952 | 82.6% | 17.4% |
| 500 nM | 0.717 | 62.2% | 37.8% |
| 1 µM | 0.437 | 37.9% | 62.1% |
| 5 µM | 0.25 | 21.7% | 78.3% |
| 10 µM | 0.113 | 9.8% | 90.2% |

## Claims

1. Use of a compound having the general formula: 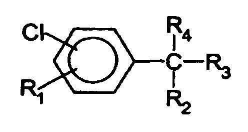 wherein R1 is selected from the group consisting of hydrogen, hydroxyl and halogen; wherein R2 is selected from the group consisting of nothing, hydrogen, phenyl and substituted phenyl, the substitution being a hydroxyl group; wherein R3 is selected from the group consisting of hydrogen, hydroxyl, lower alkyl, and lower alkoxy; wherein R4 is selected from the group consisting of sulfur-CH₂-R5, oxygen-CH₂-R5,=N-O-CH₂-R5, oxygen-phenyl-CH=CH₂, CH₂-C(CH₃)H-S-substituted phenyl, phenyl, and substituted phenyl, the substitution being selected from the group consisting of a hydroxyl group and a halogen; wherein R5 is selected from the group consisting of vinyl, phenyl, halogen mono-substituted phenyl, halogen di-substituted phenyl, phenyl-S-phenyl, CH₂-O-phenyl, CH₂-O-(halogen substituted)phenyl, and: 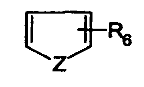 (wherein Z is sulfur, oxygen, or nitrogen); and R6 is selected from the group consisting of hydrogen and halogen,
in the manufacture of a medicament for treatment of sickle cell disease.

2. A use as claimed in claim 1, wherein said compound is selected from the following:

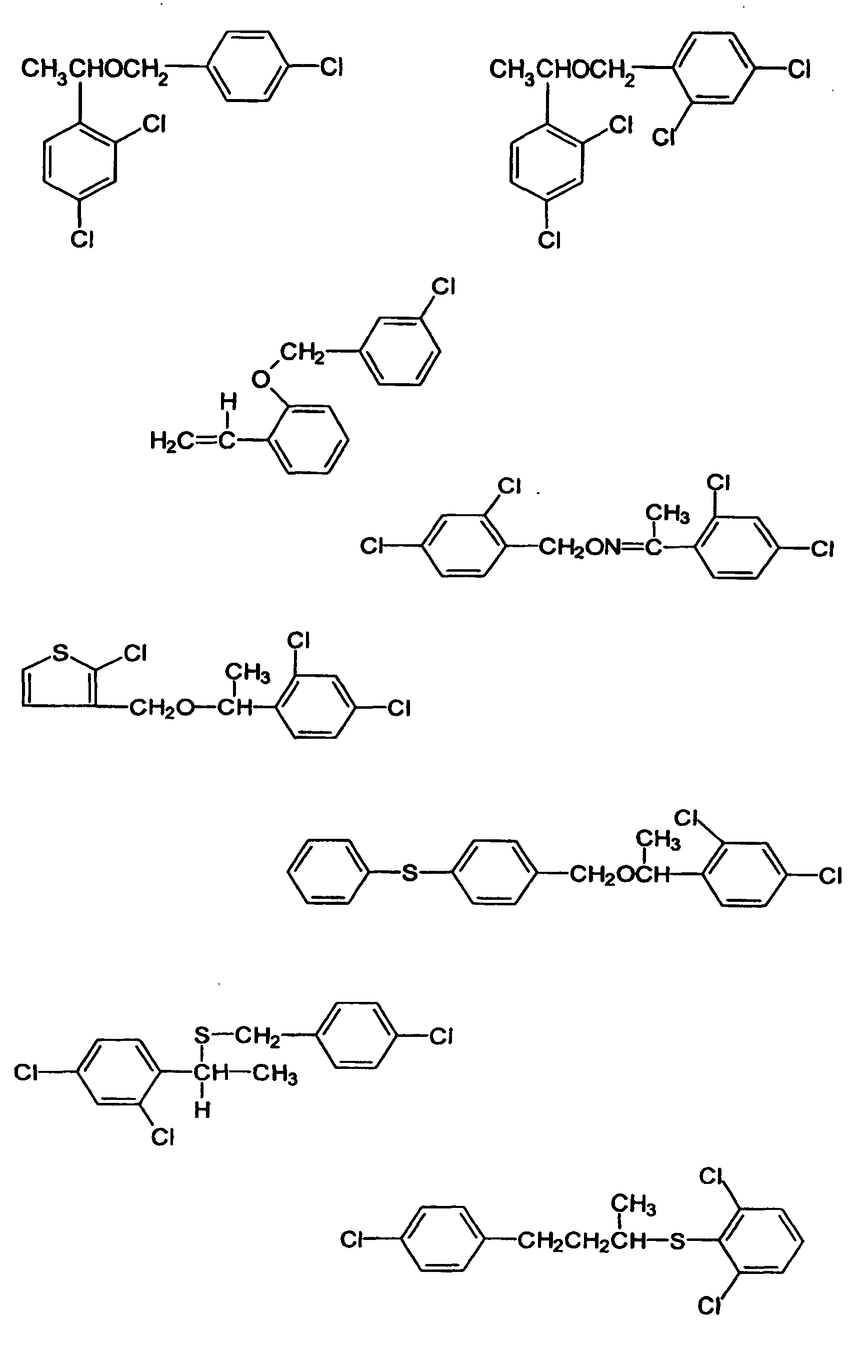

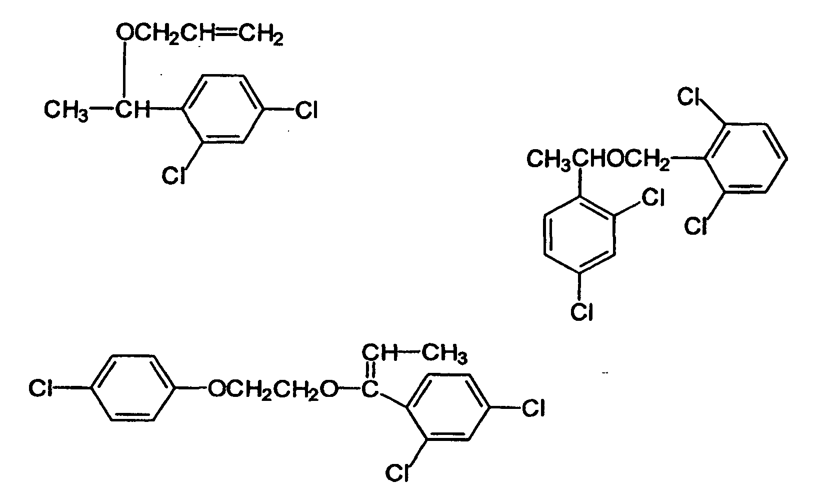

3. A use as claimed in claim 1, wherein said compound is a clotrimazole derivative and is selected from the following:

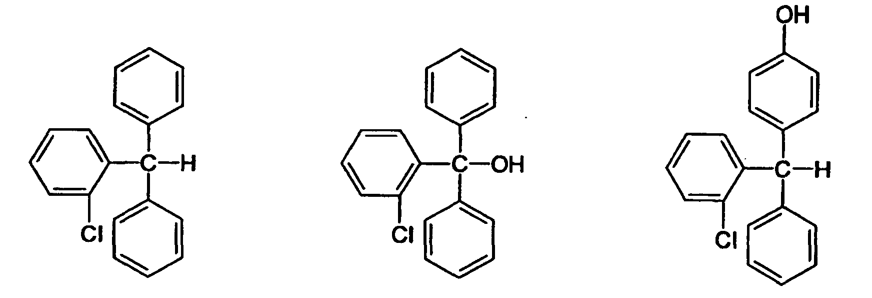

4. A use as claimed in claim 1, wherein said compound is a clotrimazole derivative and has the following structure:

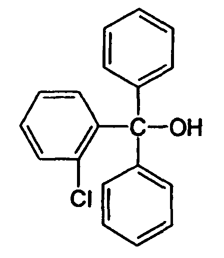

5. A use as claimed in any one of the preceding claims, wherein the treatment comprises reducing sickle erythrocyte dehydration and delaying the occurrence of erythrocyte sickling by administering to the sickle erythrocytes an amount of the molecule to induce inhibition of the Ca²⁺ -activated potassium channel at the membranes of said erythrocytes, such that sickle erythrocyte dehydration is reduced and the occurrence of erythrocyte sickling is delayed.

6. A use as claimed in any one of the preceding claims, wherein said medicament is an oral parenteral, intravenous, subcutaneous or transmucosal medicament.

7. A method for reducing sickle erythrocyte dehydration and delaying the occurrence of erythrocyte sickling, said method comprising the steps of:
administering an effective amount of at least one compound to the sickle erythrocytes ex-vivo or in-vitro, the compound having the general formula: 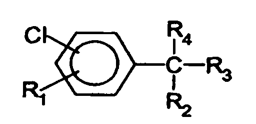
wherein R1 is selected from the group consisting of hydrogen, hydroxyl and halogen; wherein R2 is selected from the group consisting of nothing, hydrogen, phenyl and substituted phenyl, the substitution being a hydroxyl group; wherein R3 is selected from the group consisting of hydrogen, hydroxyl, lower alkyl, and lower alkoxy; wherein R4 is selected from the group consisting of sulfur-CH₂-R5, oxygen-CH₂-R5,=N-O-CH₂-R5, oxygen-phenyl-CH=CH₂, CH₂-C(CH₃)H-S-substituted phenyl, phenyl, and substituted phenyl, the substitution being selected from the group consisting of a hydroxyl group and a halogen; wherein R5 is selected from the group consisting of vinyl, phenyl, halogen mono-substituted phenyl, halogen di-substituted phenyl, phenyl-S-phenyl, CH₂-O-phenyl, CH₂-O-(halogen substituted)phenyl, and: 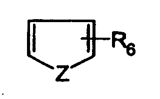 (wherein Z is sulfur, oxygen, or nitrogen); and R6 is selected from the group consisting of hydrogen and halogen; and
allowing said administered compound to induce inhibition of the Ca²⁺-activated potassium channel at the membranes of said sickle erythrocytes such that sickle erythrocyte dehydration is reduced and the occurrence of erythrocyte sickling is delayed.

8. A method as claimed in claim 7, wherein said compound is selected from the following:

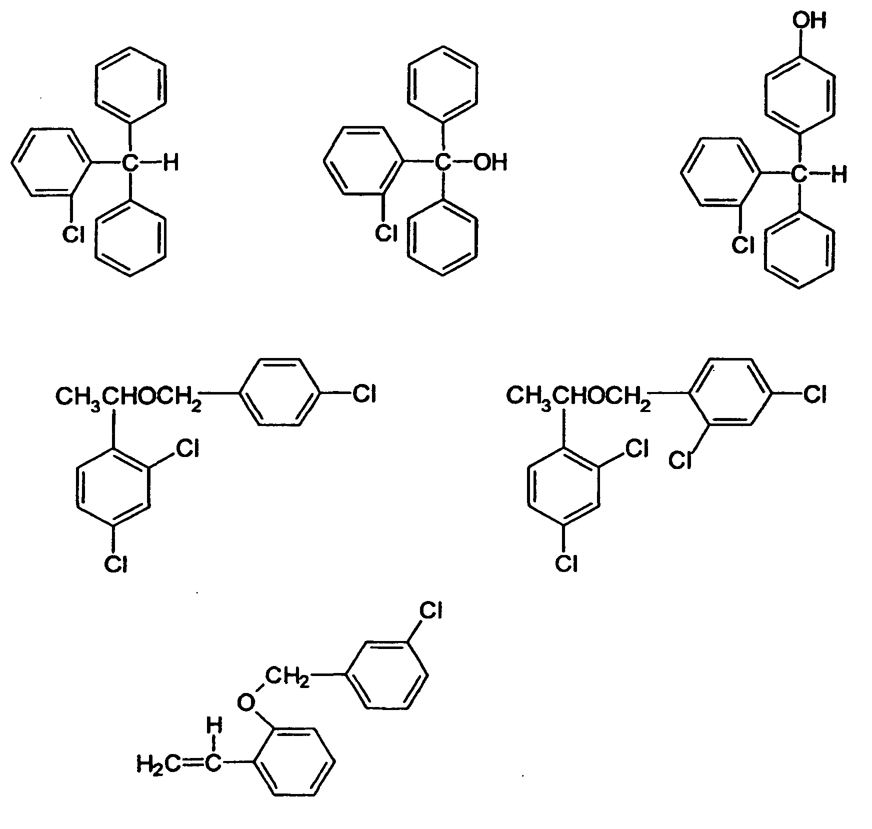

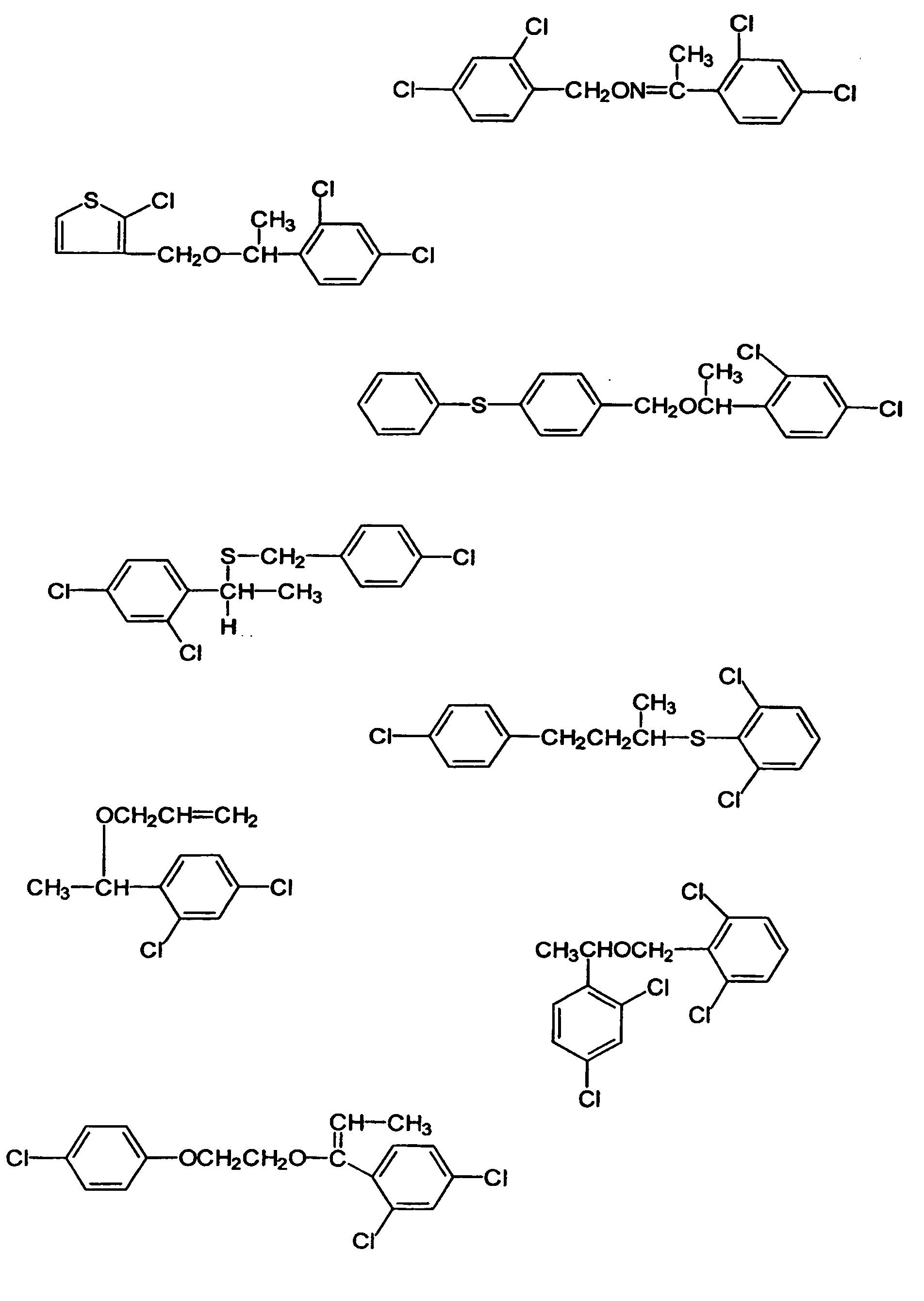

9. A method as claimed in claim 7, wherein said compound is a metabolic derivative of clotrimazole and has the following structure:

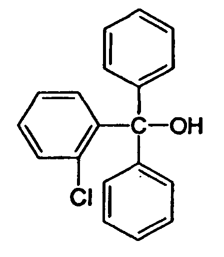

## Patentansprüche

1. Verwendung einer Verbindung mit der allgemeinen Formel: 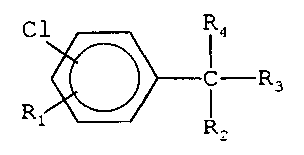 wobei R1 ausgewählt ist aus der Gruppe, bestehend aus Wasserstoff, Hydroxyl und Halogen; wobei R2 ausgewählt ist aus der Gruppe, bestehend aus nichts, Wasserstoff, Phenyl und substituiertem Phenyl, wobei die Substitution eine Hydroxylgruppe ist; wobei R3 ausgewählt ist aus der Gruppe, bestehend aus Wasserstoff, Hydroxyl, Niederalkyl und Niederalkoxy; wobei R4 ausgewählt ist aus der Gruppe, bestehend aus Schwefel-CH₂-R5, Sauerstoff-CH₂-R5, =N-O-CH₂-R5, Sauerstoff-Phenyl-CH=CH₂, CH₂-C (CH₃) H-S- (substituiertes Phenyl), Phenyl und substituiertem Phenyl, wobei die Substitution ausgewählt ist aus der Gruppe, bestehend aus einer Hydroxylgruppe und einem Halogen; wobei R5 ausgewählt ist aus der Gruppe, bestehend aus Vinyl, Phenyl, einfach mit Halogen substituiertem Phenyl, zweifach mit Halogen substituiertem Phenyl, Phenyl-S-phenyl, CH₂-O-phenyl, CH₂-O-(halogensubsituiertes Phenyl), und: 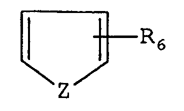 (wobei Z Schwefel, Sauerstoff oder Stickstoff ist); und R6 ausgewählt ist aus der Gruppe, bestehend aus Wasserstoff und Halogen, zur Herstellung eines Medikaments zur Behandlung von Sichelzellenkrankheit.

2. Verwendung nach Anspruch 1, wobei die Verbindung aus den folgenden ausgewählt ist:

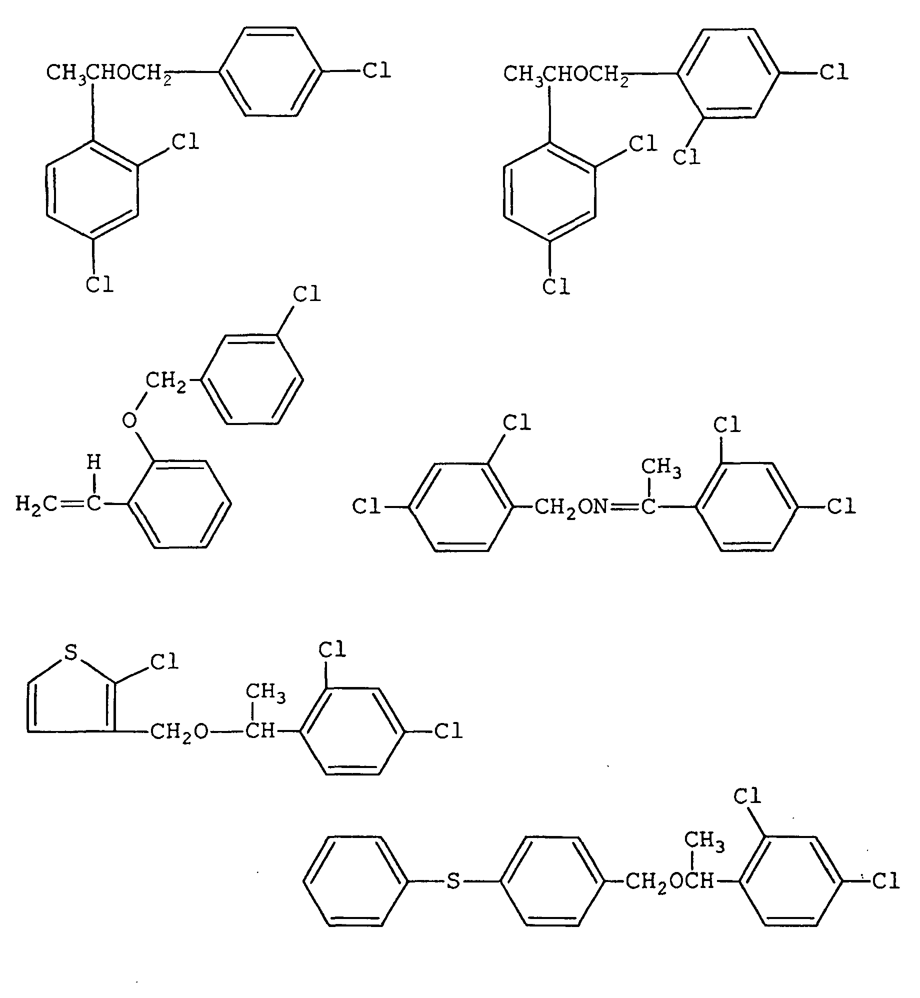

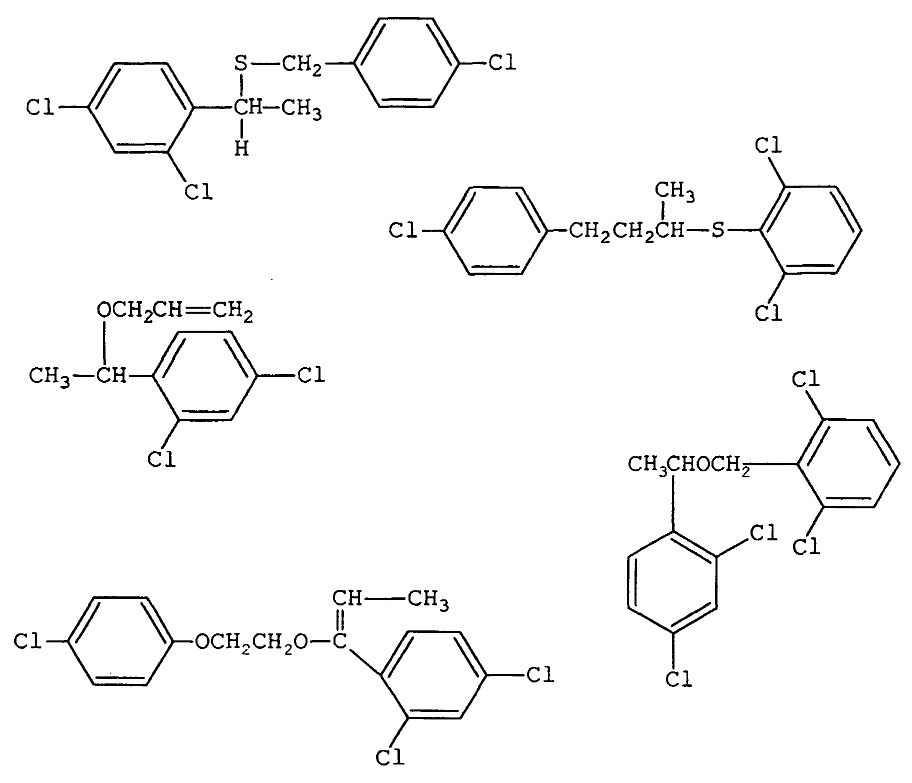

3. Verwendung nach Anspruch 1, wobei die Verbindung ein Clotrimazol-Derivat ist und aus den folgenden ausgewählt ist: OH

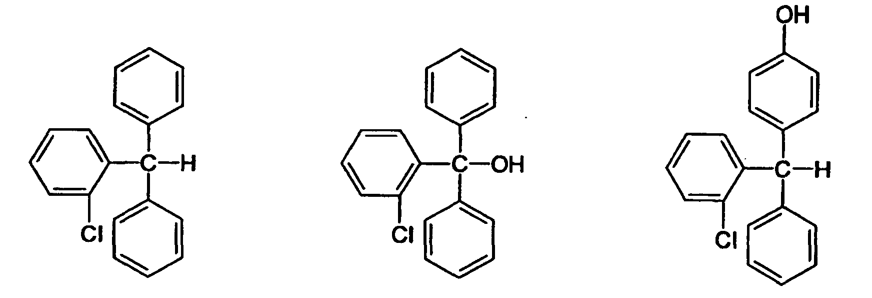

4. Verwendung nach Anspruch 1, wobei die Verbindung ein Clotrimazol-Derivat ist und die folgende Struktur aufweist:

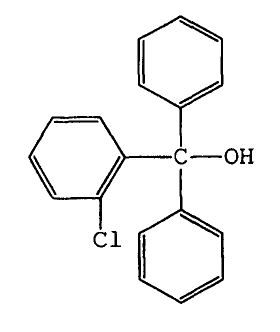

5. Verwendung nach einem der vorhergehenden Ansprüche, wobei die Behandlung die Verminderung der Sichelerythrozyten-Dehydratation und die Verzögerung des Auftretens von Erythrozytensichelung durch Verabreichung einer Menge des Moleküls an die Sichelerythrozyten umfasst, um die Hemmung der Ca²⁺-aktivierten Kaliumkanäle an den Membranen der Erythrozyten zu induzieren, so dass die Sichelerythrozyten-Dehydratation vermindert ist und das Auftreten der Erythrozytensichelung verzögert ist.

6. Verwendung nach einem der vorhergehenden Ansprüche, wobei das Medikament ein orales parenterales, intravenöses, subkutanes oder transmukosales Medikament ist.

7. Verfahren zur Verminderung der Sichelerythrozytendehydratation und Verzögerung des Auftretens der Erythrozytensichelung, wobei das Verfahren die Schritte umfasst des: Verabreichens einer wirksamen Menge mindestens einer Verbindung an die Sichelerythrozyten ex-vivo oder in-vitro, wobei die Verbindung die allgemeine Formel: 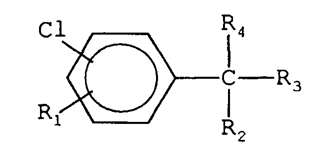 aufweist, wobei R1 ausgewählt ist aus der Gruppe, bestehend aus Wasserstoff, Hydroxyl und Halogen; wobei R2 ausgewählt ist aus der Gruppe, bestehend aus nichts, Wasserstoff, Phenyl und substituiertem Phenyl, wobei die Substitution eine Hydroxylgruppe ist; wobei R3 ausgewählt ist aus der Gruppe, bestehend aus Wasserstoff, Hydroxyl, Niederalkyl und Niederalkoxy; wobei R4 ausgewählt ist aus der Gruppe, bestehend aus Schwefel-CH₂-R₅, Sauerstoff-CH₂-R₅, =N-O-CH₂-R₅, Sauerstoff-Phenyl-CH=CH₂, CH₂-C(CH₃)H-S-(substituiertes Phenyl), Phenyl und substituiertem Phenyl, wobei die Substitution ausgewählt ist aus der Gruppe, bestehend aus einer Hydroxylgruppe und einem Halogen; wobei R5 ausgewählt ist aus der Gruppe, bestehend aus Vinyl, Phenyl, einfach mit Halogen substituiertem Phenyl, zweifach mit Halogen substituiertem Phenyl, Phenyl-S-phenyl, CH₂-O-phenyl, CH₂-O-(halogensubsituiertes Phenyl), und: 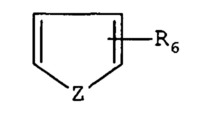 (wobei Z Schwefel, Sauerstoff oder Stickstoff ist); und R6 ausgewählt ist aus der Gruppe, bestehend aus Wasserstoff und Halogen,
Induzierenlassens der Hemmung der Ca²⁺-aktivierten Kaliumkanäle an den Membranen der Sichelerythrozyten durch die verabreichte Verbindung, so dass die Sichelerythrozyten-Dehydratation vermindert ist und das Auftreten der Erythrozytensichelung verzögert ist.

8. Verfahren nach Anspruch 7, wobei die Verbindung aus den folgenden ausgewählt ist:

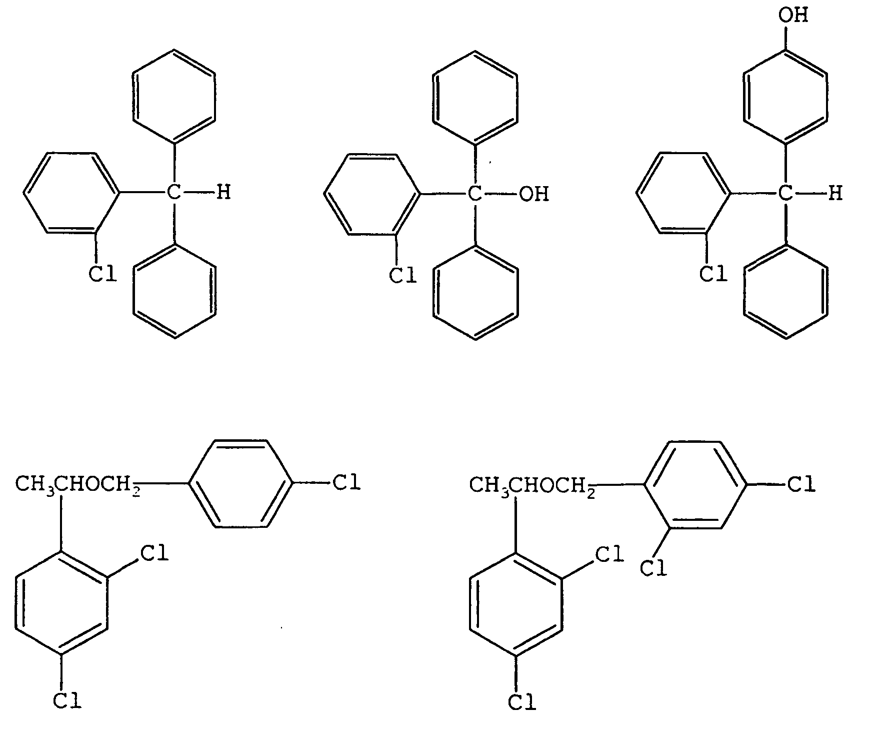

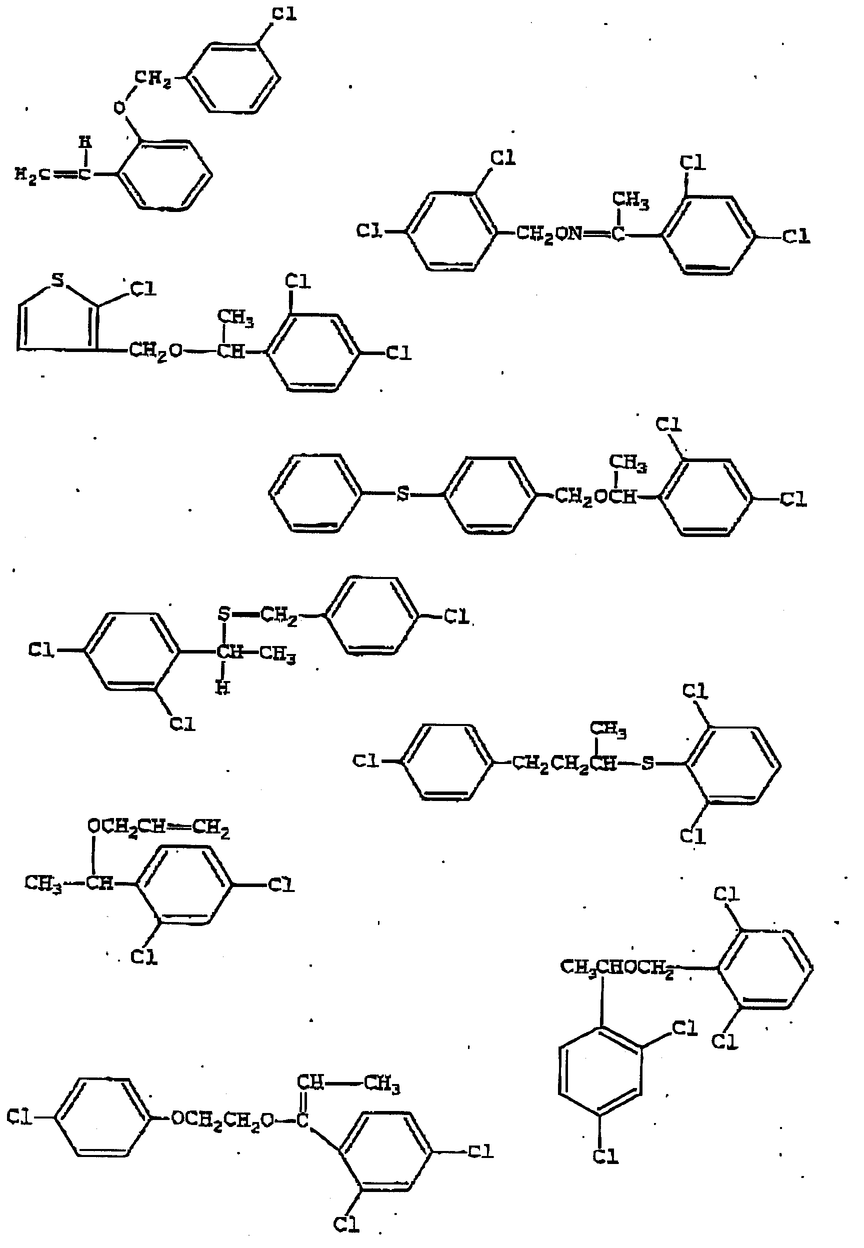

9. Verfahren nach Anspruch 7, wobei die Verbindung ein metabolisches Derivat von Clotrimazol ist und die folgende Struktur aufweist:

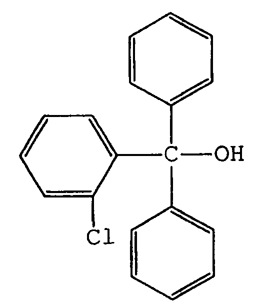

## Revendications

1. Utilisation d'un composé répondant à la formule générale : 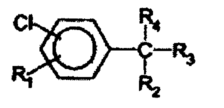 dans laquelle R1 est choisi dans le groupe consistant en un atome d'hydrogène, un groupe hydroxyle, un atome d'halogène ; R2 est choisi dans le groupe consistant en rien, un atome d'hydrogène, un groupe phényle et un groupe phényle substitué, le substituant étant un groupe hydroxyle ; R3 est choisi dans le groupe consistant en un atome d'hydrogène, un groupe hydroxyle, un groupe alkyle inférieur et un groupe alkoxy inférieur ; R4 est choisi dans le groupe consistant en des groupes soufre-CH₂-R5, oxygène-CH₂-R5, =N-O-CH₂-R5, oxygène-phényl-CH=CH₂, CH₂-C(CH₃)H-S-phényle substitué, phényle et phényle substitué, le substituant étant choisi dans le groupe consistant en un groupe hydroxyle et un atome d'halogène ; R5 est choisi dans le groupe consistant en des groupes vinyle, phényle, phényle à monosubstituant halogéno, phényle à disubstituant halogéno, phényl-S-phényle, CH₂-O-phényle, CH₂-O-phényl (à substituant halogéno), et : 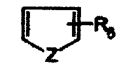 (où Z représente un atome de soufre, d'oxygène ou d'azote) ; et R6 est choisi dans le groupe consistant en un atome d'hydrogène et un atome d'halogène,
dans la production d'un médicament destiné au traitement de la maladie à hématies falciformes.

2. Utilisation suivant la revendication 1, dans laquelle ledit composé est choisi parmi les suivants :

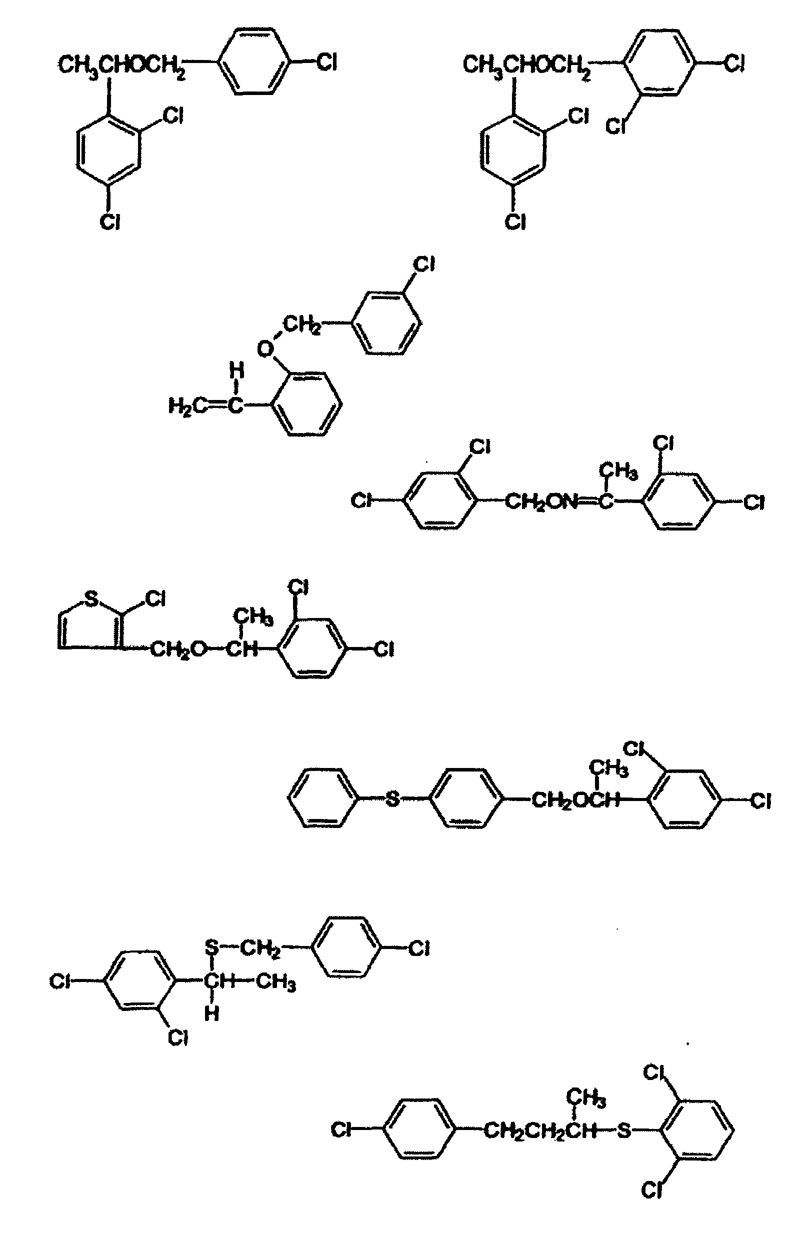

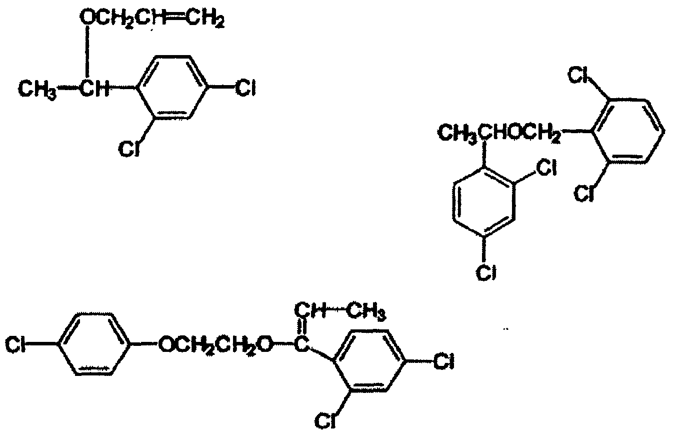

3. Utilisation suivant la revendication 1, dans laquelle ledit composé est un dérivé de clotrimazole et est choisi parmi les suivants :

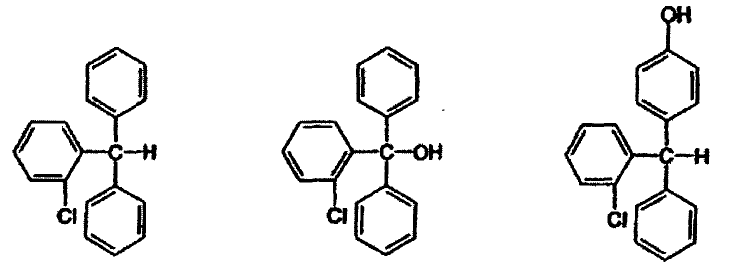

4. Utilisation suivant la revendication 1, dans laquelle ledit composé est un dérivé de clotrimazole et a la structure suivante :

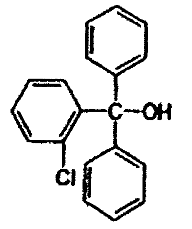

5. Utilisation suivant l'une quelconque des revendications précédentes, dans laquelle le traitement comprend la réduction de la déshydratation des érythrocytes falciformes et le retard de l'apparition de la falciformation des érythrocytes en administrant aux érythrocytes falciformes une quantité de la molécule pour réduire l'inhibition du canal potassium activé par Ca²⁺ au niveau des membranes desdits érythrocytes, de telle sorte que la déshydratation des érythrocytes falciformes soit réduite et que l'apparition de la falciformation des érythrocytes soit retardée.

6. Utilisation suivant l'une quelconque des revendications précédentes, dans laquelle ledit médicament est un médicament oral, parentéral, intraveineux, sous-cutané ou transmuqueux.

7. Méthode pour réduire la déshydratation des érythrocytes falciformes et retarder l'apparition de la falciformation des érythrocytes, ladite méthode comprenant les étapes consistant :
à administrer une quantité efficace d'au moins un composé aux érythrocytes falciformes ex vivo ou in vitro, le composé répondant à la formule générale : 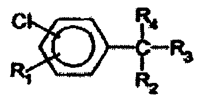
dans laquelle R1 est choisi dans le groupe consistant en un atome d'hydrogène, un groupe hydroxyle et un atome d'halogène ; R2 est choisi dans le groupe consistant en rien, un atome d'hydrogène, un groupe phényle, un groupe phényle substitué, le substituant étant un groupe hydroxyle ; R3 est choisi dans le groupe consistant en un atome d'hydrogène, un groupe hydroxyle, un groupe alkyle inférieur et un groupe alkoxy inférieur ; R4 est choisi dans le groupe consistant en des groupes soufre-CH₂-R5, oxygène-CH₂-R5, =N-O-CH₂-R5, oxygène-phényl-CH=CH₂, CH₂-C(CH₃)H-S-phényle substitué, phényle et phényle substitué, le substituant étant choisi dans le groupe consistant en un groupe hydroxyle et un atome d'halogène ; R5 est choisi dans le groupe consistant en des groupes vinyle, phényle, phényle à monosubstituant halogéno, phényle à disubstituant halogéno, phényl-S-phényle, CH₂-O-phényle, CH₂-O-phényl (à substituant halogéno), et : 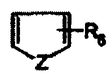 (où Z représente un atome de soufre, d'oxygène ou d'azote) ; et R6 est choisi dans le groupe consistant en un atome d'hydrogène et un atome d'halogène ; et
à laisser ledit composé administré induire l'inhibition du canal potassium activé par Ca²⁺ au niveau des membranes desdits érythrocytes falciformes de telle sorte que la déshydratation des érythrocytes falciformes soit réduite et l'apparition de la falciformation des érythrocytes soit retardée.

8. Méthode suivant la revendication 7, dans laquelle ledit composé est choisi parmi les suivants :

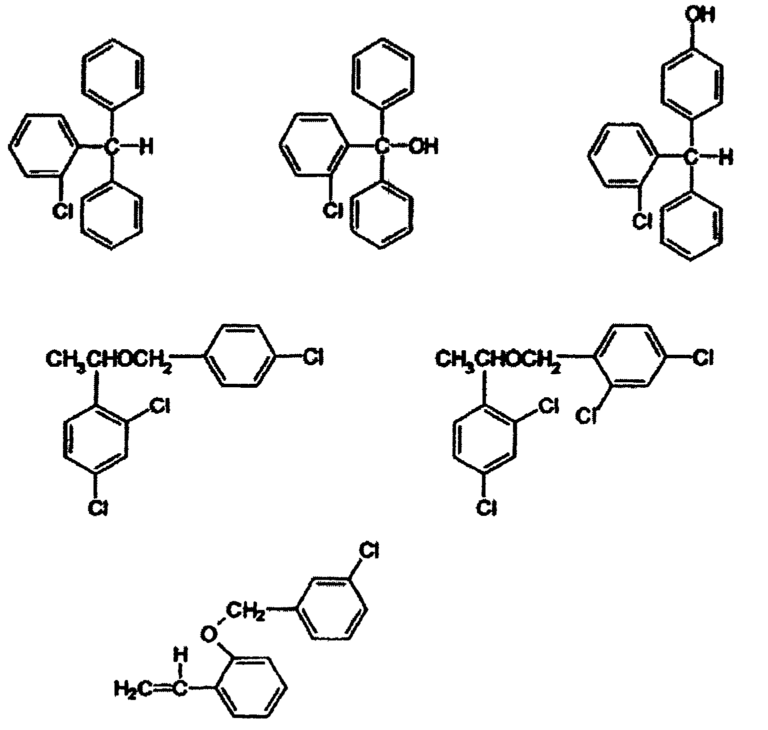

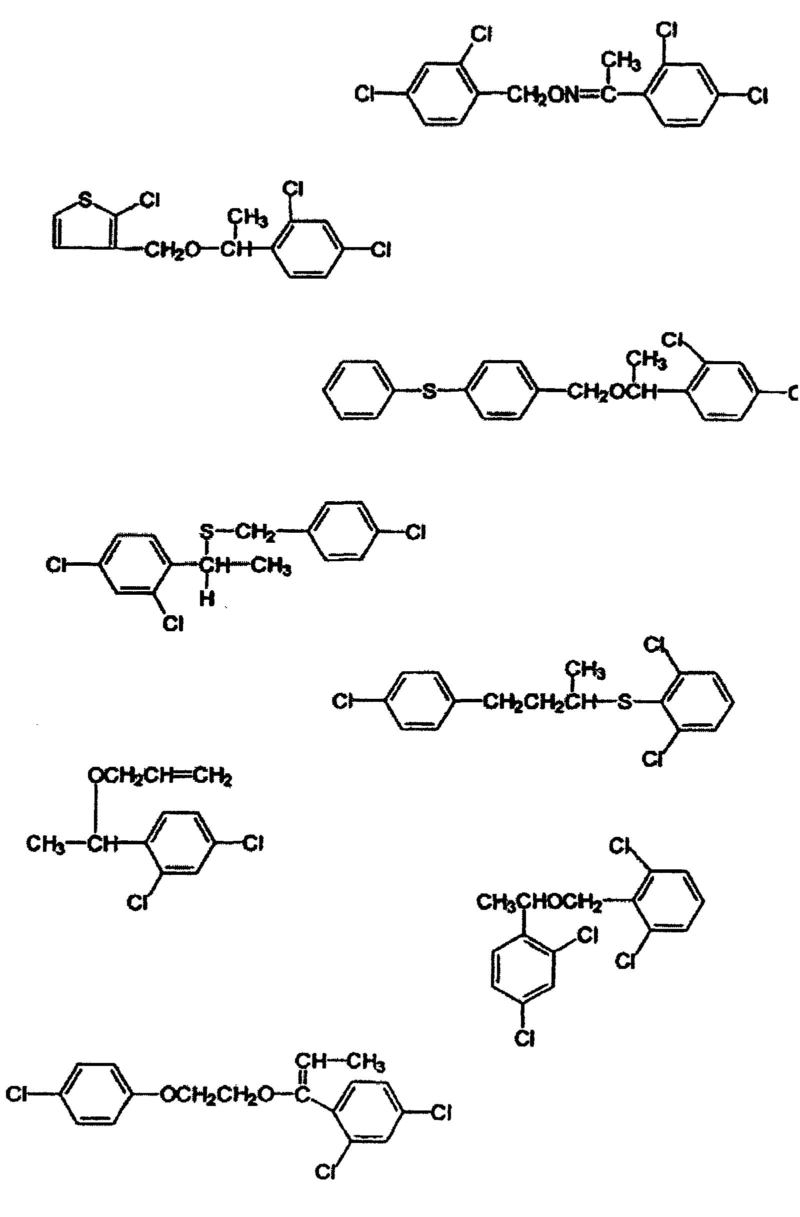

9. Méthode suivant la revendication 7, dans laquelle ledit composé est un dérivé métabolique de clotrimazole et a la structure suivante :
